# EUROPEAN PATENT SPECIFICATION

(11) **EP 4 090 407 B1**
(45) Date of publication and mention of the grant of the patent: **12.08.2026**
(21) Application number: 21741876.3
(22) Date of filing: 14.01.2021
(51) Int. Cl.: A61M 25/09

(54) **DEVICES AND METHODS FOR POSITIONING A GUIDEWIRE**
VORRICHTUNGEN UND VERFAHREN ZUR POSITIONIERUNG EINES FÜHRUNGSDRAHTES
DISPOSITIFS ET PROCÉDÉS DE POSITIONNEMENT D'UN FIL-GUIDE

(30) Priority: 15.01.2020 US 202062961440 P
(43) Date of publication of application: 23.11.2022
(73) Proprietor: Ancora Heart, Inc., Santa Clara, CA 95054 (US)
(72) Inventor: KOTMEL, Rob, Burlingame, California 94010 (US)
(74) Representative: Cooley (UK) LLP
(86) International application number: PCT/US2021/013485
(87) International publication number: WO 2021/146460

(56) References cited:
- US-A- 4 798 586
- US-A1- 2002 128 678
- US-A1- 2010 125 244
- US-A1- 2012 071 857
- US-A1- 2015 306 358
- US-A1- 2016 220 785
- US-A1- 2019 015 645
- US-A1- 2019 015 645
- US-A1- 2019 336 729

## Description

### CROSS-REFERENCE TO RELATED APPLICATIONS

### TECHNICAL FIELD

This invention relates generally to devices for positioning a guidewire

### BACKGROUND

In many medical procedures such as minimally-invasive procedures, guidewires are typically navigated through body lumens (e.g., vasculature). For example, many cardiovascular procedures such as stent placements, cardiac valve procedures, etc. typically require placement of one or more guidewires that assist in the advancement and positioning of other devices (e.g., catheters). However, guidewire placement can be challenging to perform properly. For example, a guidewire must be flexible enough to navigate potentially tortuous paths; however, this flexibility also makes it more difficult to position the guidewire with precise control. Certain kinds of procedures with dynamically moving targets, such as cardiac procedures involving a beating heart, may pose even greater challenges for guidewire placement.

Conventional guidewire placement systems typically include many components to enable precise control of the guidewire, and these complex systems are difficult to use and require great skill to operate successfully. Thus, there is a need for new and improved devices for positioning a guidewire.

### SUMMARY

The invention is according to claim 1. Preferred embodiments of the invention are set forth in the dependent claims. Described herein are devices for positioning a guidewire, such as in a patient. In some variations, a device for positioning a guidewire comprises a catheter lumen, an inner shaft arranged at least partially within the catheter lumen and comprising a shaft lumen configured to receive a guidewire, and a locking mechanism configured to lock the guidewire relative to the inner shaft to maintain at least a predetermined longitudinal offset distance between the guidewire and the catheter. The inner shaft and the catheter may be engageable so as to limit proximal longitudinal movement of the inner shaft within the catheter lumen. Furthermore, in some variations, a distal end of the catheter may be curved. In the description certain references are made to non-SI units (inches). These are to be translated to corresponding SI units (mm) using the factor: 1 inch = approximately 25.4 mm.

In some variations, the inner shaft and/or the catheter may comprise one or more features to define a proximal limit of longitudinal movement of the inner shaft within the catheter lumen. For example, the inner shaft may comprise a first feature and the catheter may comprise a second feature, where the first feature and the second feature may be engageable to at least partially define the proximal limit of longitudinal movement of the inner shaft within the catheter lumen. The first feature, the second feature, or both may comprise a stop. For example, in some variations the first feature in the inner shaft may comprise a first projection extending radially outward, and the second feature in the catheter may comprise a second projection extending radially inward to interfere with (e.g., abut) the first projection. In some variations, the first projection may comprise an open (e.g., partial) ring or closed ring arranged around a portion of the inner shaft, and the second projection may comprise a wall configured to abut the open or closed ring.

The locking mechanism may, in some variations, comprise a shaft housing (e.g., handle) coupled to the inner shaft. In these variations, the guidewire positioning device may further comprise a collet configured to tighten the shaft housing around the inner shaft and the guidewire, thereby clamping the guidewire to the inner shaft.

In some variations, a method, which is not part of the invention, for positioning a guidewire comprises arranging an inner shaft at least partially within a catheter lumen of a catheter such that the inner shaft is positioned at a proximal limit of longitudinal movement of the inner shaft relative to the catheter, advancing a guidewire into a shaft lumen of the inner shaft until the guidewire is located at a predetermined longitudinal offset distance relative to the catheter, locking the advanced guidewire relative to the inner shaft while the inner shaft is at the proximal limit of longitudinal movement, and positioning the guidewire at least in part by advancing the inner shaft.

In some variations, the arranging the inner shaft at least partially within the catheter lumen may comprise engaging a first feature of the inner shaft with a second feature of the catheter. The first feature may, for example, be arranged on a distal portion of the inner shaft, and the second feature may be arranged on a proximal portion of the catheter, where the first feature and the second feature may be engaged (e.g., abut one another) to define the proximal limit of longitudinal movement of the inner shaft. For example, the first feature, the second feature, or both may comprise a stop.

Advancing the guidewire may, in some variations, comprise advancing the guidewire until a distal end of the guidewire extends distally beyond a distal end of the catheter, such as by the predetermined longitudinal offset distance. Once advanced to such a position, the guidewire may be locked while the inner shaft is at the proximal limit of longitudinal movement (while the distal end of the guidewire extends distally beyond the distal end of the catheter), thereby substantially preventing the distal end of the guidewire from moving proximally into the catheter.

The guidewire may be locked relative to the inner shaft in any suitable manner. For example, in some variations, the inner shaft may be coupled to a shaft housing (e.g., a handle), and locking the guidewire relative to the inner shaft may comprise compressing the shaft housing around the inner shaft and the guidewire. For example, compressing the shaft housing may comprise tightening a collet around at least a portion of the shaft housing.

After advancing and locking the guidewire, the guidewire may be moved by moving the inner shaft. In some variations, positioning the guidewire may comprise alternately advancing the inner shaft and the catheter, thereby providing support and/or protection for the guidewire with the catheter during guidewire positioning. Alternately advancing the inner shaft and the catheter may, for example, comprise advancing the inner shaft until a distal end of the guidewire encounters an obstruction, advancing the catheter to reduce the distance between the distal end of the guidewire and a distal end of the catheter, and further advancing the inner shaft. During alternate advancement of the inner shaft and the catheter, at least the predetermined longitudinal offset distance between the guidewire and the catheter may be maintained. In other words, the guidewire may be substantially prevented from being withdrawn fully into the catheter. In some variations, after the guidewire and the guidewire positioning device are placed at a location, the guidewire may be unlocked and withdrawn from the inner shaft through the shaft lumen. At least a portion of the rest of the guidewire positioning device may remain, and in some variations the method, which is not part of the invention, may further comprise advancing a second guidewire into the guidewire positioning device (e.g., to facilitate placement of one or more other catheter devices to the same location, such as over the second guidewire). Alternatively, in some variations the guidewire positioning device may be withdrawn, leaving the guidewire to remain at the location (e.g., to facilitate placement of one or more other catheter devices to the same location, such as over the guidewire).

The devices described herein may be used in any suitable application or procedure involving guidewire placement. In an exemplary variation, the devices may be used to advance a catheter into a ventricle of a heart (e.g., left ventricle). The devices may, for example, be used to advance a catheter into a heart ventricle for reshaping (e.g., reverse-modeling) a heart ventricle. In this example, the method, which is not part of the invention, may comprise advancing the catheter into a left ventricle of a heart, and positioning the guidewire may comprise alternately advancing the inner shaft and the catheter around a subvalvular space of a mitral valve behind chordae tendineae of the left ventricle.

### BRIEF DESCRIPTION OF THE DRAWINGS

FIGS. 1A-1H depict schematic representations of exemplary variations of a device for positioning a guidewire.
FIG. 2 depicts a flowchart representation of one exemplary variation of a method, which is not part of the invention, for positioning a guidewire.
FIGS. 3A-3F depict a schematic representation of a method, which is not part of the invention, for positioning a guidewire.
FIG. 4A depicts a proximal portion of an exemplary variation of a guidewire positioning device, with an inner shaft of the device at a distal portion of its range of longitudinal motion.
FIG. 4B depicts a proximal portion of the guidewire positioning device depicted in FIG. 4A, with an inner shaft of the device at a proximal portion of its range of longitudinal motion.
FIG. 4C depicts a partial detailed view of the guidewire positioning device depicted in FIG. 4A.
FIG. 5A is a side view of a distal portion of an exemplary variation of a guidewire positioning device.
FIG. 5B is another side view of the distal portion of the guidewire positioning device depicted in FIG. 5A.
FIG. 6 depicts an exemplary variation of a guide catheter device.
FIGS. 7A and 7B depict a schematic representation of a portion of an exemplary variation of a method, which is not part of the invention, for positioning a guidewire.
FIGS. 8A-8E depict schematic representations of exemplary variations of a locking mechanism in a guidewire positioning device.

### DETAILED DESCRIPTION

Examples of various aspects and variations of the invention are described herein and illustrated in the accompanying drawings. The following description is not intended to limit the invention to these embodiments, but rather to enable a person skilled in the art to make and use this invention.

Described herein are exemplary variations of devices for positioning (e.g., advancing) a guidewire. In some variations, the devices for positioning a guidewire may be used to navigate a guidewire in a patient (e.g., in cardiovasculature such as a chamber of the heart, in a blood vessel, etc.). As described below, the devices described herein may enable a user to advance a guidewire in a reliable, safe, and easy-to-use manner. For example, the catheter device may provide simpler navigational control and support of a guidewire arranged in a lumen of the catheter device, and/or help prevent undesirable withdrawal or retraction of the guidewire into the catheter device during advancement of the guidewire in a patient which would otherwise interfere with the positioning process. These features may, for example, increase procedural simplicity and safety during guidewire positioning.

### Devices for positioning a guidewire

In some variations, a device for positioning a guidewire may comprise a catheter comprising a catheter lumen, an inner shaft arranged at least partially within the catheter lumen and comprising a shaft lumen configured to receive a guidewire, and a locking mechanism configured to lock the guidewire relative to the inner shaft. The inner shaft and the catheter may be engageable so as to limit longitudinal movement in at least one direction (e.g., limit proximal longitudinal movement) of the inner shaft within the catheter lumen. In some variations, the locking mechanism may be configured to lock the guidewire relative to the inner shaft in a manner that maintains at least a predetermined longitudinal offset distance between the guidewire and the catheter.

The catheter may comprise an elongate body with a proximal end and a distal end. The catheter may further comprise a catheter lumen within the elongate body and be configured to receive therein the inner shaft so as to permit longitudinal and/or rotational motion of the inner shaft within the catheter. Generally, the elongate body may be sized to fit and be advanced within a body passageway, such as vasculature and/or cardiac chambers (e.g., left ventricle or right ventricle). The catheter may comprise a catheter housing (e.g., handle) which may be coupled (e.g., bonded with epoxy, coupled through one or more suitable fasteners and/or mechanical interfit, etc.) to a proximal portion of the catheter, such that longitudinal and/or rotational motion of the catheter housing may effect longitudinal and/or rotational motion of at least a portion of the elongate body. The catheter housing may, for example, be formed (e.g., molded) with an ergonomic and/or frictional grip to enable easy handling.

The distal end of the catheter may, in some variations, have a curved shape which may be designed for easier navigation around curved surfaces (e.g., ventricular tissue, vasculature) and/or atraumatic advancement. For example, when a guidewire is being advanced from within the catheter, the curved distal end of the catheter may be positioned to point away from tissue, to help prevent perforation of the tissue by the guidewire as the guidewire is being extended or advanced out of the catheter. Furthermore, the distal tip of the catheter may increase in diameter and terminate in a rounded tip without sharp edges, to improve atraumatic characteristics of the device. However, various shapes and dimensions of the catheter may depend at least in part on the intended application of the guidewire positioning device. For example, in some variations in which the guidewire positioning device is used to position a guidewire in a left ventricle, the catheter in the guidewire positioning device may have a length sufficient to extend at least from a femoral arterial access point, into the left ventricle, around the subvalvular space, and out of the outflow tract to optionally re-cross the aortic arch of a patient.

The inner shaft may comprise an elongate body with a proximal end and a distal end, and may be arranged within the catheter lumen (e.g., in a telescopic manner). The inner shaft may further comprise a shaft lumen configured to receive a guidewire, and permit longitudinal and/or rotational motion of the guidewire therein. Additionally, a shaft housing (e.g., handle) may be coupled (e.g., bonded with epoxy, coupled with one or more suitable fasteners and/or mechanical interfit, etc.) to a proximal portion of the inner shaft, such that longitudinal and/or rotational motion of the shaft housing may effect longitudinal and/or rotational motion of the inner shaft within the catheter.

The locking mechanism may be configured to lock the position of the guidewire relative to the inner shaft. For example, after the guidewire is locked relative to the inner shaft, longitudinal and/or rotational motion of the inner shaft (e.g., via the shaft housing coupled to the proximal portion of the inner shaft) may effect longitudinal and/or rotational motion of the guidewire within the guidewire positioning device. In some variations, the locking mechanism may comprise a shaft housing that is coupled to a portion of the inner shaft, where the shaft housing may be tightened around the inner shaft and the guidewire to thereby lock the guidewire relative to the inner shaft through a compressive force. For example, the shaft housing may be engageable with a collet nut or other mechanism that clamps around a portion of the shaft housing to tighten the shaft housing around the inner shaft and guidewire, and lock or otherwise fix the inner shaft and guidewire together. The collet nut may, for example, engage the shaft housing with threads or in any suitable manner. However, the locking mechanism may comprise any suitable structure for fixing the guidewire relative to the inner shaft (e.g., pinch-type clamps, etc.).

In some variations, the guidewire positioning device may further comprise one or more stop features configured to limit longitudinal movement of the inner shaft within the catheter lumen. For example, in some variations, the catheter and/or the inner shaft (and/or another structure coupled thereto) may comprise one or more stops. In some variations, the inner shaft may comprise a first feature, and the catheter may comprise a second feature engageable with the first feature, where the first feature and/or the second feature comprises a stop that provides a proximal limit of longitudinal movement of the inner shaft within the catheter lumen. For example, the inner shaft may comprise at least one projection extending radially outward (e.g., outer ring), and the catheter may comprise at least one projection extending radially inward (e.g., inner ring or suitable wall), where the radially outward projection and the radially inward projection may abut one another to limit travel. In an exemplary variation, a distal portion of the inner shaft may comprise at least one projection that is configured to abut at least one projection at a proximal portion of the catheter at the proximal limit of the inner shaft's range of longitudinal motion. Additionally or alternatively, a limit of longitudinal movement may be formed by any suitable stop features (e.g., tethers, bands, other tension devices, magnets, etc.) positioned at a preset desired location from the distal end of the catheter (or sized, such as with a preset desired length of a tension device, to limit the position of the inner shaft relative to the catheter).

As further described herein, in some variations, the guidewire may be locked when a distal end of the guidewire is extending distally beyond a distal end of the catheter by a predetermined longitudinal offset distance, while the inner shaft is at a proximal limit of longitudinal movement within the catheter lumen. In these variations, longitudinal and/or rotational movement of the inner shaft may control longitudinal and/or rotational movement of the locked guidewire, but the locked guidewire may be substantially prevented from having its distal end be fully retracted into the catheter lumen, thereby improving safety and ease-of-use while positioning the guidewire in a patient.

In some variations, the guidewire may be retracted into the catheter lumen (e.g., to permit removal of the guidewire from the catheter through the proximal portion of the catheter) by unlocking the guidewire from the inner shaft. For example, the locking mechanism may be disengaged or otherwise unlocked, to thereby unlock the guidewire from the inner shaft. However, additionally or alternatively, in some variations the stop may be a "soft stop" that may be overcome with a sufficiently large force, or disengaged (or otherwise removed) with a clutch (e.g., lever, latch, etc.) in the event full retraction of the guidewire is desirable (e.g., without unlocking the guidewire from the inner shaft). Even further, in some variations the stop may additionally or alternatively be movable between a stopping configuration in which the stop limits motion of the inner shaft, and a permissive configuration in which the stop does not limit motion of the inner shaft. The stop may be toggled between the stopping configuration and the permissible configuration with, for example, a lever, latch, squeezing mechanism, or any suitable mechanism.

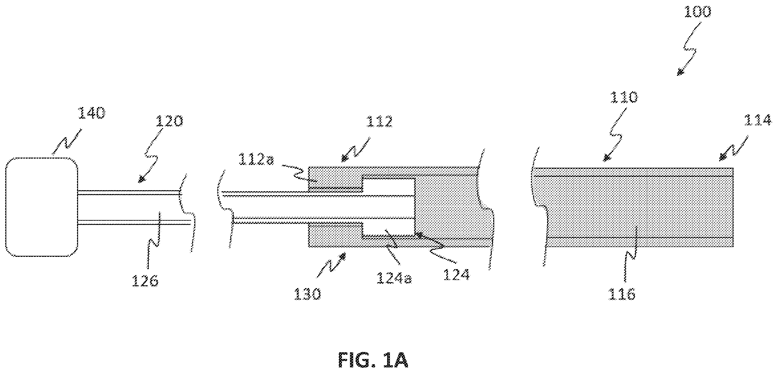

FIG. 1A depicts a schematic representation of an exemplary variation of a device 100 for positioning a guidewire. The guidewire positioning device 100 may comprise a catheter 110 comprising a catheter lumen 116 extending between a proximal portion 112 and a distal portion 114, and an inner shaft 120 arranged at least partially within the catheter lumen 116 comprising a shaft lumen 126 configured to receive a guidewire (not shown). The inner shaft 120 may have a limited range of longitudinal travel within the catheter lumen 116, where the travel of the inner shaft 120 may be limited by a proximal limit of longitudinal motion and/or a distal limit of longitudinal motion. The guidewire positioning device 100 may further comprise a locking mechanism 140 configured to lock the guidewire relative to the inner shaft, such that motion (e.g., longitudinal and/or rotational positioning) of the guidewire may be controlled by a user operating the inner shaft via a connected handle or the like.

In some variations, the guidewire positioning device 100 may comprise one or more stops 130 configured to limit longitudinal movement of the inner shaft 120 within the catheter lumen 116. The stop(s) 130 may comprise one or more features of the inner shaft 120 and/or the catheter lumen 116. For example, as shown in FIG. 1A, the catheter 110 may comprise a stop feature 112a at a proximal portion 112 of the catheter 110, and the inner shaft 120 may comprise a stop feature 124a at a distal portion 124 of the inner shaft 120. Generally, when the inner shaft 120 is moved proximally, the inner shaft stop feature 124a may engage (e.g., abut) the catheter stop feature 112a, thereby preventing the inner shaft 120 from moving farther proximally.

In some variations, the catheter stop feature 112a may comprise one or more radially inward projections. The one or more radially inward projections may be integrally formed (e.g., molded) with at least a portion of the catheter 110, or may be formed separately and subsequently coupled to the catheter 110 (e.g., coupled to an inner surface of the catheter lumen 116 with an adhesive such as epoxy, with threads, with an interference fit, etc.). In some variations, the inner shaft stop feature 124a may comprise one or more radially outward projections which may be integrally formed with the inner shaft 120 or formed separately and subsequently coupled to the inner shaft 120 (e.g., coupled to an outer surface of the inner shaft 120 with an adhesive, threads, interference fit, etc.).

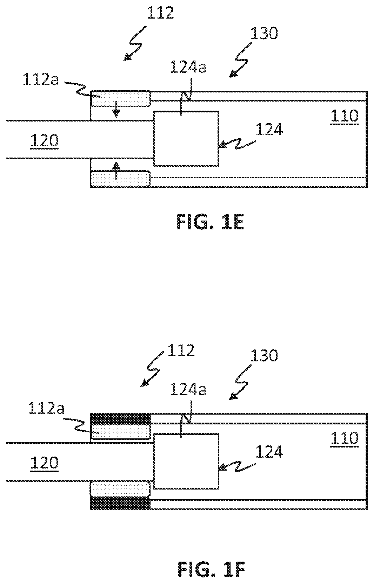

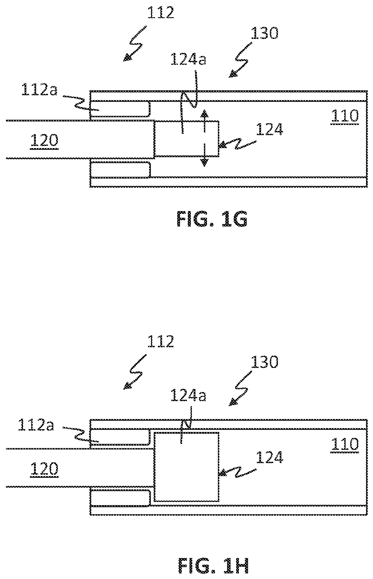

In some variations, the stop 130 may be selectively transitioned between a stopping configuration in which the stop limits motion of the inner shaft, and a permissive configuration in which the stop does not limit motion of the inner shaft. The stop 130 may toggle between the stopping configuration and the permissive configuration in any suitable manner. For example, the radial projections in the catheter and/or inner shaft may be selectively movable in a radially inward and/or outward direction (e.g., slidable in a radial direction such as along a track or slot, or radially inflatable and/or deflatable, etc.), such as with a lever, latch, squeezing operation, or the like. To transition the stop 130 into the stopping configuration, the one or more radial projections in the catheter stop feature 112a may be controlled to move radially inward, and/or the one or more radial projections in the inner shaft stop feature 124a may be controlled to move radially outward, so as to create physical interference between the stop features 112a and 124a. For example, in some variations, such as that shown in FIGS. 1E and 1F, the radial projections in the catheter stop feature 112a may be controlled to move radially inward to transition from a permissive configuration (FIG. 1E) to a stopping configuration (FIG. 1F). Additionally or alternatively, such as that shown in FIGS. 1G and 1H, the radial projections in the inner shaft stop feature 124a may be controlled to move radially outward to transition from a permissive configuration (FIG. 1G) to a stopping configuration (FIG. 1H).

Conversely, to transition the stop 130 into the permissive configuration, the one or more radial projections in the catheter stop feature 112a may be controlled to move radially outward, and/or the one or more radial projections in the inner shaft stop feature 124a may be controlled to move radially inward, so as to create physical clearance between the stop features 112a and 124a. For example, with reference to FIGS. 1E and 1F, the radial projections in the catheter stop feature 112a may be controlled to move radially outward to transition from the stopping configuration (FIG. 1F) to the permissive configuration (FIG. 1E). Additionally or alternatively, with reference to FIGS. 1G and 1H, the radial projections in the inner shaft stop feature 124a may be controlled to move radially inward to transition from the stopping configuration (FIG. 1H) to the permissive configuration (FIG. 1G). It should be understood that in some variations, the radial projection(s) in both the stop features 112a and 124a may be movable in such a manner, and in other variations it may be sufficient that the radial projection(s) in solely either the catheter stop feature 112a or inner shaft stop feature 124a may be movable, in order to toggle between a stopping configuration and a permissive configuration.

The catheter stop feature(s) 112a and inner shaft stop feature(s) 124a may have any suitable shape and structure for engaging and forming a stop 130. For example, as shown in FIG. 1A, the catheter stop feature(s) 112a and/or the inner shaft stop feature(s) 124a may comprise a ring, partial ring, fingers (e.g., equally or unequally spaced apart) which may project radially inward (e.g., for the catheter stop feature 112a) or radially outward (e.g., for the inner shaft stop feature 124a). As shown in FIG. 1A, the abutting surfaces of the stop features 112a and 124a may be generally orthogonal to the longitudinal axis of the catheter lumen 116 and inner shaft 120.

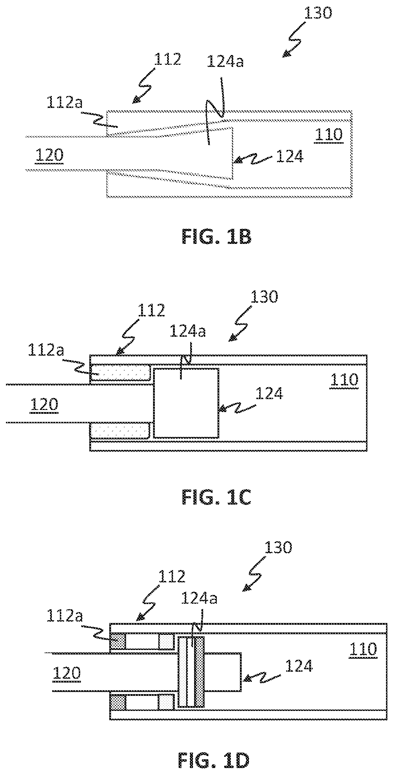

In some variations, the abutting surfaces of the stop features 112a and 124a may be at any suitable angle. For example, FIG. 1B shows an exemplary variation of a stop 130 comprising sloped or tapered abutting surfaces. As shown in FIG. 1B, the proximal portion 112 of the catheter 110 may comprise a narrowing, funnel-like stop feature 112a that is configured to engage a correspondingly flared stop feature 124a on the distal portion 124 of the inner shaft 120. When the inner shaft 120 is withdrawn proximally, the sloped inner shaft stop feature 124a abuts or otherwise engages the catheter stop feature 112a, thereby prevent further proximal movement of the inner shaft 120 relative to the catheter 110.

Additionally or alternatively, in some variations such as that shown in FIG. 1C, the stop 130 may comprise a compressible material or other suitable deformable structure. For example, one or both of the stop features 112a and 124a may comprise a compressible material or other suitable deformable structure. Like the variations shown in FIGS. 1A and 1B, the inner shaft stop feature 124a may be configured to abut the catheter stop feature 112a so as to limit further proximal movement of the inner shaft 120 relative to the catheter 110. However, in the exemplary variation shown in FIG. 1C, the stop 130 may be a "soft stop" in that the proximal limit may be overcome if the inner shaft 120 is moved proximally with sufficient proximal force. While the compressible material is shown in FIG. 1C to be in the catheter stop feature 112a, it should be understood that in some variations the compressible material may additionally or alternatively be in the inner shaft stop feature 124a. The compressible material may, for example, comprise foam, epoxy, an inflatable cushion or balloon, and/or the like. In some variations in which the compressible material comprises an inflatable structure, the structure may be selectively inflated and/or deflated to transition the stop between a stopping configuration and a permissive configuration, similar to that described above.

In some variations, the stop 130 may comprise engagement between the inner shaft 120 and the catheter 110 that does not involve mechanical interference. FIG. 1D illustrates an exemplary variation of a stop 130 comprising one or more magnets or magnetic material, in which magnetic repulsion may limit longitudinal travel of the inner shaft 120 in the catheter 110. For example, the inner shaft stop feature 124a may comprise a magnet or magnetic material, and the catheter stop feature 112a may similarly comprise a magnet or magnetic material. The stop features 112a and 124a may be mutually magnetically repulsive, such that as the inner shaft 120 is withdrawn proximally (and the inner shaft stop feature 124a approaches the catheter stop feature 112a), the magnetic repulsion resists further proximal movement of the inner shaft 120. For example, one or more of the stop features 112a and/or 124a may comprise a permanent magnet or magnetic material, an electromagnetic material, etc.

The stops described herein (e.g., with respect to FIGS. 1A-1D) for limiting longitudinal movement of the inner shaft may be combined in any suitable manner. For example, the compressible material described above with respect to FIG. 1C may have a tapered shape similar to that shown in FIG. 1B. As another example, any of the stop variations such as those involving mechanical interference (e.g., described above with respect to FIGS. 1A-1C) may further comprise magnetic material to supplement the stop with magnetic repulsion.

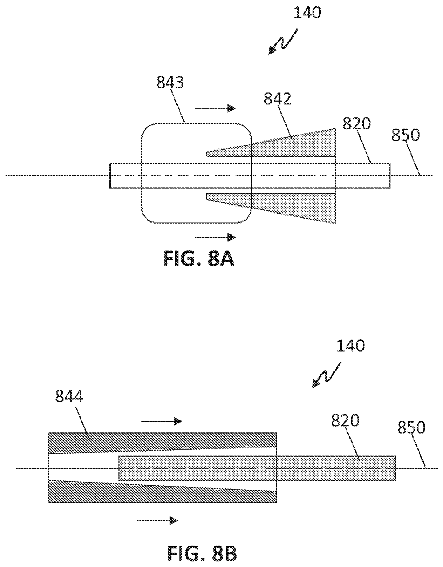

The guidewire positioning device 100 may further comprise a locking mechanism 140 configured to lock a guidewire in the shaft lumen to the inner shaft. After locking the guidewire to the inner shaft via the locking mechanism 140, the guidewire may be controlled by moving the inner shaft within the catheter. The locking mechanism 140 may, for example, clamp or wedge the guidewire against the inner shaft and/or a feature (e.g., handle or hub) fixed to the inner shaft. The locking mechanism 140 may be arranged at or near a proximal portion of the inner shaft, so as to be accessible outside a patient's body when the distal portion of the guidewire positioning device is in the patient. In some variations, the locking mechanism 140 may comprise a shaft housing (e.g., handle) that is compressible over the inner shaft and guidewire contained therein, to thereby lock the guidewire and the inner shaft to move (e.g., longitudinally and/or rotationally) together. As shown in FIG. 8A, the shaft housing or other portion of the locking mechanism 140 may, for example, comprise a collet mechanism 842 that may be radially compressed with a collet nut 843, a sleeve, or any suitable mechanism. The collet mechanism 842 may, for example, be arranged around a proximal portion of the inner shaft (e.g., accessible outside a patient's body when the distal portion of the guidewire positioning device is in the patient) and configured to be compressed with the collet nut 843.

The locking mechanism 140 may additionally or alternatively include any suitable variations of locking features. In another example as shown in FIG. 8B, the locking mechanism 140 may comprise a longitudinally movable member 844 such as a slidable or rolling wedge member that is longitudinally movable (e.g., distally and proximally) between a locked position and an unlocked position, where in the locked position the slidable or rolling wedge member compresses the inner shaft (or housing fixedly coupled thereto) against the guidewire 850, thereby locking the guidewire and inner shaft to move together. The longitudinally movable member 844 and/or the inner shaft 820 may comprise one or more detents or the like to help secure the member 844 in the locked position or the unlocked position. In some variations, the longitudinally moveable member 844 may be spring-loaded to be biased toward the locked position (and e.g., temporarily retracted to the unlocked position to open the inner shaft lumen to allow for guidewire advancement into the inner shaft).

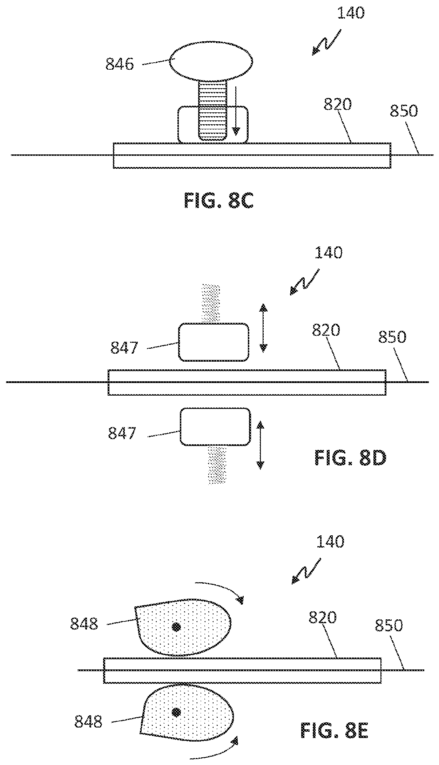

As another example as shown in FIG. 8C, the locking mechanism 140 may comprise a laterally movable member 846 (e.g., thumb screw, set screw, other threaded member, etc.) that may be movable in a radial direction to clamp or wedge the inner shaft (or housing fixedly coupled thereto) to the guidewire to lock the guidewire and inner shaft together. Multiple laterally movable members 846 may be arranged circumferentially around the inner shaft to clamp upon the guidewire in multiple radial directions, and these multiple laterally movable members may be controlled independently or in synchrony through mating features, etc.

As yet another example as shown in FIG. 8D, the locking mechanism 140 may comprise one or more compression spring-loaded members 847 that may be biased to clamp upon the inner shaft 820. When the guidewire 850 is loaded into the inner shaft 820, the spring-loaded members 847 may be retracted (e.g., by depressing a lever or other suitable mechanism) to open the inner shaft lumen and allow advancement of the guidewire 850, then the spring-loaded members 847 may be released to clamp and lock the guidewire into place, thereby locking the guide wire and inner shaft together. Although the spring-loaded members 847 are shown as laterally-movable members in FIG. 8D, it should be understood that in other variations the spring-loaded members 847 may alternatively be longitudinally movable. Furthermore, although two members 847 are shown in FIG. 8D, it should be understood that in other variations any suitable number of spring-loaded members may be arranged circumferentially around the inner shaft, and may be controlled independently or in synchrony, similar to that described above.

As yet another example as shown in FIG. 8E, the locking mechanism 140 may comprise one or more rotatable cam mechanisms 848 with variable radius, such that rotation of the cam mechanisms to a locking rotational position (e.g., with a longer radius directed transverse to the inner shaft 820) may cause the cam mechanisms to pinch or otherwise compress the inner shaft 820 against the guidewire 850, thereby locking the guidewire and inner shaft together. Similar to that described above, any suitable number of cam mechanisms may be arranged around the inner shaft 820, and may be movable independently or in synchrony. Furthermore, in some variations the cam mechanisms may be spring-loaded to bias the locking mechanism toward the locking rotational positions.

Any of the above-described locking mechanisms 140 may be used in combination together and/or with any other suitable locking mechanism for locking the inner shaft and guidewire to be movable together.

### Methods, which are not part of the invention, for positioning a guidewire

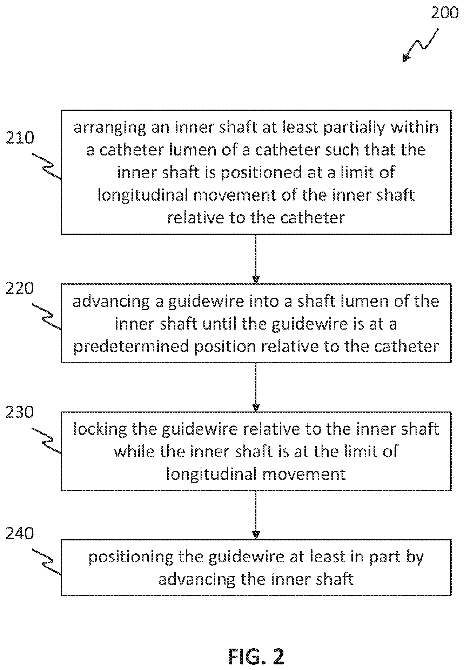

FIG. 2 depicts a flowchart representation of an exemplary method, which is not part of the invention, 200 for positioning a guidewire. The method, which is not part of the invention, 200 may comprise arranging an inner shaft (210) at least partially within a catheter lumen of a catheter such that the inner shaft is positioned at a limit (e.g., proximal limit) of longitudinal movement of the inner shaft relative to the catheter, advancing a guidewire (220) into a shaft lumen of the inner shaft until the guidewire is at a predetermined position relative to the catheter, locking the guidewire relative to the inner shaft (230) while the inner shaft is at the limit of longitudinal movement, and positioning the guidewire at least in part by advancing the inner shaft (240). In some variations, prior to locking the guidewire relative to the inner shaft, the guidewire may be advanced to a predetermined position that is located at a predetermined longitudinal offset distance relative to the catheter. For example, the guidewire may be positioned such that a distal end of the guidewire is extending distally beyond a distal end of the catheter by a predetermined offset distance. The guidewire may be locked relative to the inner shaft while the guidewire is in this distally-extending position and the inner shaft is at a proximal limit of its longitudinal movement. In this example, longitudinal and/or rotational movement of the inner shaft, such as by a user, may control a corresponding longitudinal and/or rotational movement of the locked guidewire, but the locked guidewire may be substantially prevented from having its distal end be fully retracted into the catheter lumen, thereby improving safety and ease-of-use for positioning the guidewire in a patient.

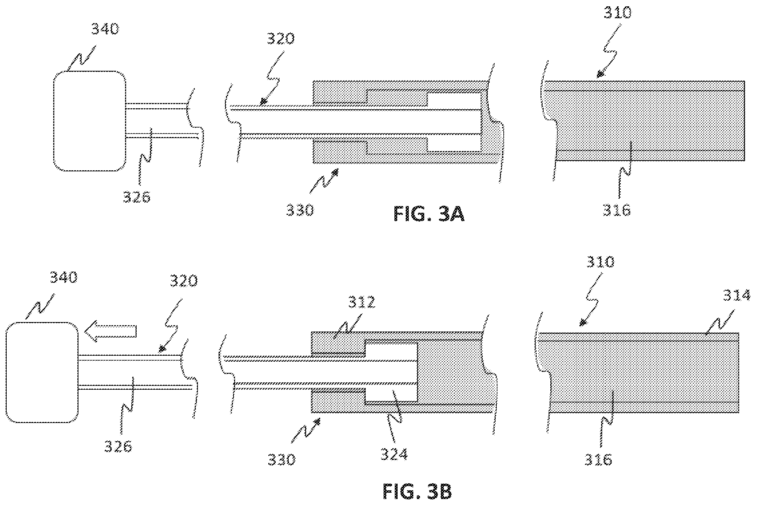

In some variations, the method 200, which is not part of the invention, may involve use of a guidewire positioning device such as that described herein. For example, for the sake of illustration only, various aspects of the method 200 are shown and described in FIGS. 3A-3F depicting the guidewire positioning device shown and described above with respect to FIG. 1A.

As shown in FIG. 3A, the method 200, which is not part of the invention, may comprise arranging an inner shaft 320 at least partially within a catheter lumen 316 of a catheter 310, where the catheter lumen 316 may, for example, extend between a proximal portion 312 and a distal portion 314. The inner shaft 320 may be configured to move longitudinally (e.g., telescopically) within the catheter lumen 316. The inner shaft 320 may further comprise a shaft lumen 326 for receiving a guidewire (not shown) to be positioned. During a procedure in which guidewire placement is desirable, a user (e.g., physician or other medical practitioner) may move the inner shaft 320 in a proximal direction until the inner shaft 320 encounters a proximal limit of its longitudinal range of motion within the catheter lumen at stop 330, as shown in FIG. 3B. For example, arranging the inner shaft may comprise engaging a first stop feature (e.g., at distal portion 324 of the inner shaft) with a second stop feature (e.g., at proximal portion 312 of the catheter). While arranging the inner shaft 320 in such a manner, a guidewire may be present within the shaft lumen 326, or the guidewire may be inserted into the shaft lumen 326 after the inner shaft 320 is positioned at its proximal limit.

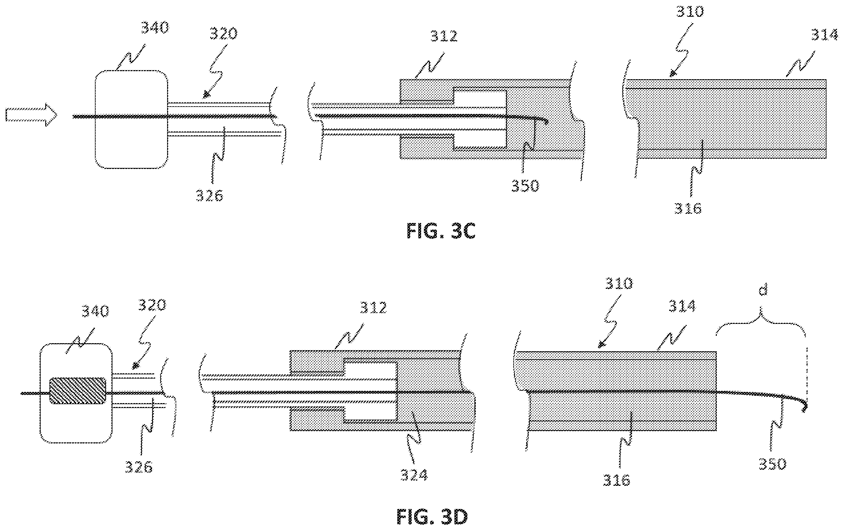

As shown in FIG. 3C, a guidewire 350 may be arranged within the inner shaft lumen 326. The guidewire may be any suitable kind of guidewire, with dimensions sufficient for the related procedure (e.g., delivery of an implant, catheter, or other suitable device). In some variations, the guidewire 350 may have an atraumatic distal end (e.g., blunted, curved such as in a "J" shape, etc.) which may, for example, reduce injury to tissue during advancement outside the catheter.

The guidewire 350 may be advanced to a predetermined desired location. For example, advancing the guidewire may comprise advancing the guidewire until a distal end of the guidewire extends distally beyond a distal end of the catheter. In some variations, the guidewire may be advanced until its distal end extends distally beyond the distal end of the catheter by a longitudinal offset distance (d), as shown in FIG. 3D. This distance (d) may be any suitable distance, such as between about 0.1 cm and about 36 cm, between about 0.1 cm and about 20 cm, between about 0.1 cm and about 15 cm, between about 0.1 cm and about 10 cm, between about 0.1 cm and about 5 cm, between about 0.1 cm and about 4 cm, between about 0.1 cm and about 3 cm, between about 1 cm and about 4 cm, between about 1 cm and about 4 cm, etc.

When the guidewire 350 is located at the predetermined desired location, the guidewire 350 may be locked to, or otherwise fixed relative to, the inner shaft 320. For example, a locking mechanism 340 may be configured to couple the guidewire 350 relative to the inner shaft, as described in further detail herein and shown schematically in FIG. 3D. In some variations, the method, which is not part of the invention, may comprise locking the guidewire relative to the inner shaft while the inner shaft is at the proximal limit and the guidewire 350 extends distally beyond the distal end of the catheter. Accordingly, in these variations, because when the inner shaft is at its proximal-most position, the guidewire (fixed relative to the inner shaft) still has its distal end extending beyond the distal end of the catheter, the locked guidewire is substantially prevented from moving proximally fully into the catheter. The locked guidewire may then be subsequently positioned as desired (e.g., in a body lumen, in other tissue such as a heart chamber, etc.) by longitudinally and/or rotationally moving (e.g., torqueing or otherwise advancing) the inner shaft, with reduced risk of the guidewire being inadvertently withdrawn into the guidewire.

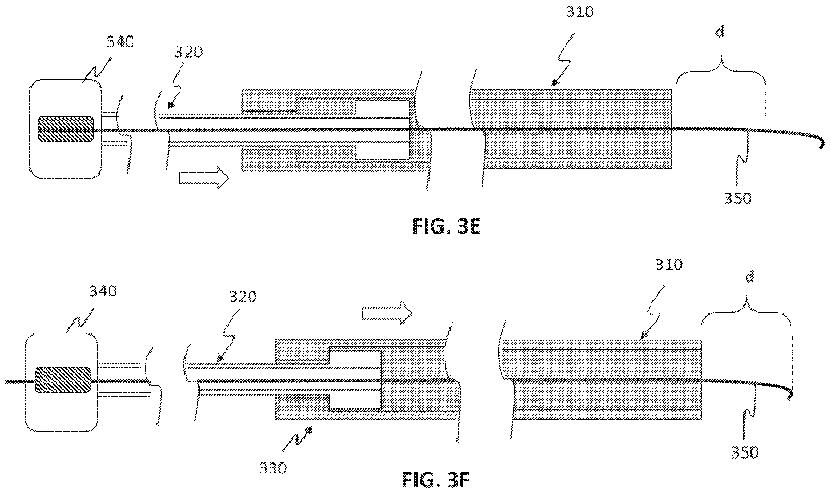

In some variations, the method, which is not part of the invention, of positioning a guidewire may comprise alternately advancing the inner shaft (with the guidewire fixed in position relative to the inner shaft as described above) and the catheter. The alternate advancement of the inner shaft and the catheter may for example, improve control of the guidewire by providing structural support to the guidewire and/or protect the guidewire by surrounding most of the guidewire as the guidewire is generally advanced. For example, FIG. 3E illustrates a configuration in which the guidewire 350 is advanced beyond the predetermined longitudinal offset distance (d) by advancing the inner shaft 320 distally. In some variations, the guidewire 350 may be advanced by another predetermined interval distance (e.g., between about 1 cm and about 5 cm), or until the guidewire 350 encounters an obstruction (e.g., an anatomical structure, such as trabeculations, muscle bands, and/or other obstacles along a ventricle wall). In the configuration shown in FIG. 3E, the guidewire 350 has moved in a distal direction, such as within a body lumen or otherwise toward a suitable target location. FIG. 3D illustrates another configuration in which the catheter 310 has been advanced, reducing the distance between the distal end of the guidewire 350 and the distal end of the catheter 310. The alternating inner shaft advancement (FIG. 3E) and catheter advancement (FIG. 3F) may be repeated as desired, to further advance the guidewire and/or catheter to desired target location(s).

In some variations, after the guidewire 350 and the catheter 310 are positioned at the desired target location(s), the guidewire 350 may be removed from the catheter 310, leaving the catheter 310 at the target location to perform any desired function of the catheter. Alternatively, the guidewire 350 may be unlocked from the inner shaft 320, and the guidewire positioning device (comprising the catheter 310 and inner shaft 320) may be removed, leaving the guidewire 350 at the target location. For example, other devices may be navigated over the placed guidewire 350 after the guidewire positioning device is removed.

Additionally or alternatively, the guidewire may be removed and replaced by a second guidewire may be inserted into the placed catheter, such as for use in guiding placement of other implants, catheters, etc. over the second guidewire. The second guidewire may, for example, have different dimensions (e.g., outer diameter) suitable for use with other catheters or other devices.

Various aspects of the method, which is not part of the invention, for positioning a guidewire are described herein with respect to FIGS. 3A-3F primarily in a particular sequential order for sake of illustration. However, it should be understood that in some variations, at least some of the steps may be performed in parallel or in another suitable order. For example, in some variations, the guidewire 350 may be advanced within the shaft lumen 326 to a predetermined location prior to arranging the inner shaft 320 at its proximal limit.

### Example

In some variations, the devices described herein may be used to position or otherwise navigate a guidewire in cardiovasculature. For example, the devices described herein may be used to position a guidewire for use in positioning one or more catheters and/or implantable devices. As described in further detail below, one illustrative example in which such guidewire positioning may be performed is a procedure for securing an implantable device to a ventricular wall for reshaping a heart ventricle. Exemplary implantable device for reshaping a heart ventricle are described in detail in U.S. Patent No. 8,343,173, U.S. Patent No. 8,641,727, U.S. Patent Application Publication No. 2018/0140421 and U.S. Patent Application Publication No. 2018/0154111. However, it should be understood that the devices for positioning a guidewire may be used or performed in conjunction with any suitable procedures, such as tricuspid, pulmonic or aortic valve repair, LAA closure, or procedures within the right ventricle.

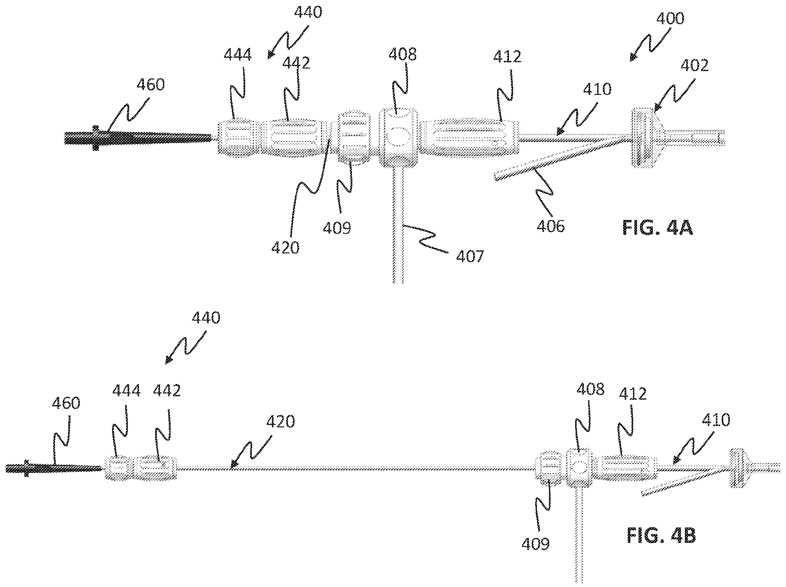

FIGS. 4A-4C depict an exemplary variation of a guidewire positioning device 400. The guidewire positioning device 400 may, for example, be used to access the subvalvular space in a left ventricle of a patient's heart, and to facilitate guidewire placement between the chordae and tendineae and the endocardium. Device 400 may comprise a catheter 410 comprising a catheter lumen, an inner shaft 420 arranged at least partially within the catheter lumen and comprising a shaft lumen configured to receive a guidewire for positioning, and a locking mechanism 440 configured to lock the guidewire relative to the inner shaft. The catheter 410 and/or inner shaft 420 may, for example, be slidably coupled such that the inner shaft 420 may longitudinally and/or rotationally move within the catheter 410. For example, FIG. 4A illustrates the device 400 in a collapsed configuration, in which the inner shaft 420 is in a more distal position within the catheter lumen. FIG. 4B illustrates the device 400 in an extended configuration, in which the inner shaft 420 is in a more proximal position within the catheter lumen. In some variations, the guidewire positioning device 400 may have a working length of between about 100 cm and about 200 cm, between about 130 cm and about 140 cm, or about 137 cm, which may be enough length to traverse the distance from a femoral artery access point into the left ventricle, around the left ventricle, out of the outflow tract, and over the aortic arch.

The catheter 410 may comprise an elongate body having a proximal portion and a distal portion (e.g., the catheter 410 may comprise a hypotube). The catheter lumen may extend within the elongate body, between the proximal portion and the distal portion of the catheter 410. In some variations, the elongate body of the catheter 410 may have a length between about 40 cm and about 50 cm, or about 45 cm, or any suitable length depending on the application. One or more markers may be arranged along the elongate body to facilitate tracking of catheter position within a patient as the guidewire positioning device is manipulated. For example, the catheter may comprise one or more radiopaque markers of a radiopaque material (e.g., platinum) that are visible under fluoroscopy, or other suitable guidance imaging. In some variations, the radiopaque markers may comprise ring or band markers encircling the elongate body at various longitudinal locations (e.g., one or more locations near the distal end 414 of the catheter 410 to help enable visualization of the catheter tip 410).

In some variations, the proximal portion (e.g., proximal end) of the elongate body may be coupled to a catheter handle 412 or other housing, so as to enable longitudinal and/or rotational control of the catheter 410. For example, the handle 412 may be coupled to the catheter 410 with epoxy or other suitable bonding agent, and/or with a mechanical interference fit, etc. As shown in FIG. 4A, the handle 412 may comprise grip elements such as ribs and/or frictional features (e.g., rubberized grip) to improve ergonomics and control of the catheter 410 using the handle 412.

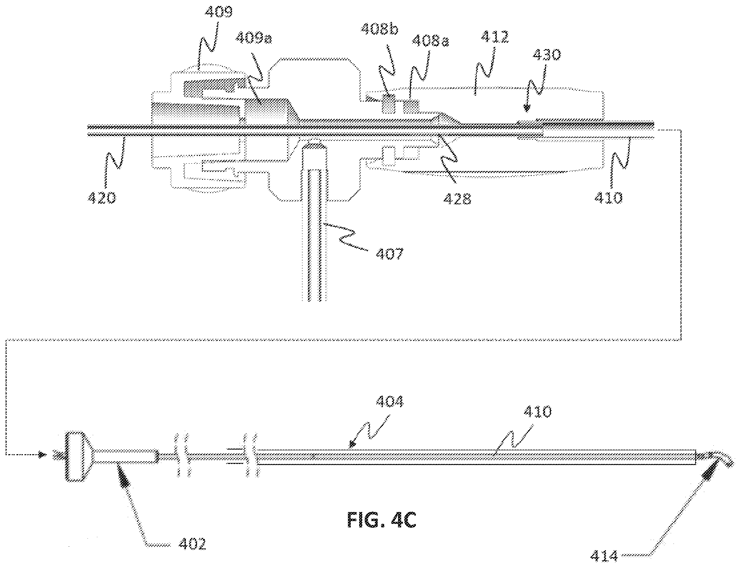

In some variations, the device 400 may comprise a hub 408 which may, for example, provide fluidic access in and/or out of the catheter 410 and/or the inner shaft 420. For example, as shown in FIG. 4C, the hub 408 may be coupled to the handle 412 (e.g., with threads, a mechanical interference fit, etc.). A flush tube 407 may be coupled to a port on the hub 408, such that fluid (e.g., saline) may be introduced into the hub 408, into one or more slots in the catheter 410 and/or inner shaft 420 (e.g., longitudinal slot cutout 428 shown in FIG. 4C), and subsequently into the catheter lumen and/or inner shaft for flushing the lumen(s) of the device 400. In some variations, as shown in the detailed view of FIG. 4C, the engagement between the hub 408 and the handle 412 may comprise at least one seal 408a (e.g., O-ring or other suitable seal) to form a fluidic seal between the hub 408 and the handle 412. Additionally or alternatively, at least one retaining ring 408b may be arranged at the interface between the hub 408 and the handle 412. In some variations, the device 400 may further comprise a hemostasis valve 409 comprising a rotational seal that may be opened and/or closed to help maintain hemostasis within the patient. For example, the hemostasis valve 409 may comprise a rotatable element threadingly coupled to a proximal portion of the hub 408, where selective rotation of the rotatable element may compress or release one or more seals 409a (e.g., silicone gasket). One or more washers (e.g., Teflon washer) or other suitable load-distributing elements may also be arranged in the hemostasis valve.

In some variations, at least a portion of the catheter 410 may be configured to be arranged within an introducer assembly 402, which may comprise one or more valves which may selectively permit trapped air to bleed out of the introducer assembly 402, such as through a prep tube 406. A proximal portion of the prep tube 406 may be positioned outside of the patient when the guidewire device 400 is in use, in order to allow air from inside the introducer assembly 402 to escape and help maintain hemostasis, for example.

The distal portion of the catheter 410 may, in some variations, comprise a distal tip region comprising one or more pre-shaped curves (e.g., curves that are pre-formed during manufacturing). For example, as shown FIG. 4C, the distal tip region may comprise one or more contours and/or bends, which may be configured to facilitate navigation of the catheter around curved tissue during catheter advancement. In some variations, the distal portion of the catheter may be pre-formed during manufacturing through a suitable process such as molding, extrusion, thermoforming, plastic deformation through bending, etc. For example, the distal portion of the catheter may be curved or contoured without active actuation by a practitioner with control wires or the like. The distal tip region may, for example, have one or more pre-shaped curves to facilitate positioning in the subannular groove (e.g., junction of left ventricular wall and mitral valve annulus) behind/around the chordae tendineae. For example, the distal tip of the catheter 410 may comprise a tip radius of curvature between about 0.3 inches and about 0.7 inches diametric, or about 0.5 inches diametric. In some variations, a tip radius of curvature that is nominal 0.48 inches diametric (e.g., with a tolerance range of about between about 0.31 inches and 0.65 inches) is configured to easily maneuver around obstacles during advancement and return out the outflow tract from the left ventricle. Furthermore, in some variations, the distal portion of the catheter may have atraumatic characteristics in that the distal tip of the catheter may be rounded and/or the distal portion of the catheter (e.g., the curved portion) may increase in diameter toward the distal end, such that the distal tip outer diameter is larger than the diameter of the rest (e.g., central or proximal portion) of the elongate body of the catheter. For example, in some variations the tip outer diameter may be about 0.110 inches and the rest of the elongate body may have an outer diameter of up to about 0.080 inches which is less than the tip outer diameter.

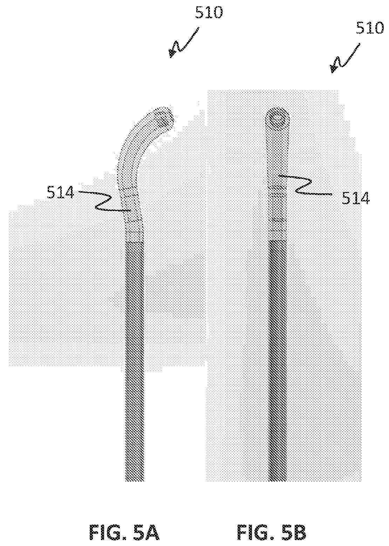

As shown in FIGS. 5A and 5B, a distal portion 514 of a catheter 510 (which may be similar to catheter 410) may comprise one or more curves generally oriented within a single plane. However, in other variations, a distal portion of the catheter may comprise one or more contours and/or bends oriented within multiple planes. In some variations, the distal portion of a catheter (e.g., catheter 410, 510) may comprise a material that is more flexible than the rest of the elongate body of the catheter, which may, for example, help reduce the risk of tissue perforation and/or other damage as the catheter is advanced within a patient. For example, while in some variations most of the elongate body of the catheter may comprise braid-reinforced PEBAX that varies in hardness from approximately Shore D 72 to Shore D 35, proximally to distally, respectively, the distal portion of the catheter itself may comprise PEBAX without braid reinforcement, having a hardness of less than or equal to about 35 D.

The inner shaft 420 may, as described herein, be slidably arranged within the catheter lumen. In some variations, the inner shaft 420 may be configured to have a longitudinal range of travel within the catheter lumen. For example, the inner shaft 420 may have a proximal limit of its longitudinal motion due to one or more stops 430 as shown in FIG. 4C. As shown in FIG. 4C, a distal portion of the inner shaft 420 may comprise an outer sleeve or other outward projection that is configured to engage with a proximal wall or other inward projection at a proximal portion of the catheter 410. In other words, the inner shaft 420 may be proximally withdrawn (FIG. 4B) until the distal portion of the inner shaft 420 interferes or stops against the proximal portion of the catheter 410. While an exemplary variation of the stop 430 is shown in FIG. 4C, it should be understood that the stop 430 may comprise a combination of any suitable features (e.g., in the catheter 410, in the inner shaft 420, in the catheter handle 412, in the hub 408, etc.) for limiting movement of the inner shaft 420. For example, any of the stops described above with respect to FIGS. 1A-1D may be included as part of device 400. In some variations, like the catheter, the inner shaft may comprise one or more markers to facilitate position of the inner shaft within the patient as the guidewire positioning device is manipulated. In some variations, one or more markers may be arranged along the inner shaft to facilitate tracking of inner shaft position within a patient as the guidewire positioning device is manipulated. For example, the inner shaft 420 may comprise one or more radiopaque markers of a radiopaque material (e.g., platinum) that are visible under fluoroscopy, or other suitable guidance imaging. In some variations, the radiopaque markers may comprise ring or band markers encircling the inner shaft at various longitudinal locations.

The inner shaft 420 may comprise an elongate body having a proximal portion and a distal portion (e.g., the inner shaft 420 may comprise a hypotube). In some variations, the elongate body of the inner shaft 420 may have a length between about 20 cm and about 30 cm, or about 25 cm, or any suitable length depending on the application. The shaft lumen may extend within the elongate body, between the proximal portion and the distal portion of the catheter 420. As described above, the shaft lumen may be configured to receive a guidewire (e.g., through a guidewire introducer 460 as shown in FIGS. 4A and 4B). Specific dimensions of the inner shaft may depend on the specific application of the device. For example, in some variations, the shaft lumen may be configured to receive a guidewire having an outer diameter of about 0.035" and/or about 0.018", with a working length compatible for placement around the subvalvular space of the ventricle into the descending aorta (e.g., from a femoral arterial access point).

In some variations, the proximal portion (e.g., proximal end) of the inner shaft elongate body may be coupled to a shaft handle 442 or other housing, so as to enable longitudinal and/or rotational control of the inner shaft 420. For example, the handle 442 may be coupled to the inner shaft 420 with epoxy or other suitable bonding agent, and/or with a mechanical interference fit, etc. As shown in FIG. 4A, the handle 442 may comprise grip elements to help improve ergonomics and/or control of the inner shaft 420.

In some variations, the device 400 may comprise a locking mechanism 440 configured to lock the position of the guidewire relative to the inner shaft 420. For example, as shown in FIG. 4A, the locking mechanism 440 may comprise a collet (e.g., a portion of the inner shaft handle 442), and a collet nut 444 configured to tighten the locking mechanism 440 around the inner shaft 420 and the guidewire positioned therewithin. For example, the collet nut 444 may be threadingly engaged with the handle 442, such that rotating the collet nut 444 urges the engagement between the collet nut 444 and handle 442 for clamping around the inner shaft 444, thereby pinching and fixing the guidewire position therewithin. Like the handle 442, the collet nut 444 may comprise a housing comprising one or more suitable grip features to help improve ergonomics and/or control of the collet nut 444. However, the device 400 may comprise any suitable locking mechanism 440 for functionally coupling actuation of the inner shaft 420 to actuation of the guidewire.

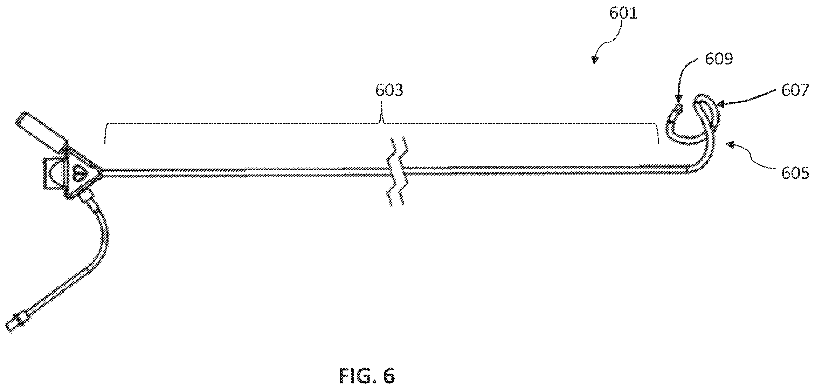

The guidewire positioning device 400 may be used in combination with one or more suitable devices. For example, as shown in the schematic of FIG. 4C, the guidewire positioning device 400 may be advanced within a guide catheter 404. Guide catheter 404 may, for example, be positioned proximate along a ventricular wall region where a device is to be implanted, and/or between the chordae tendineae and the endocardium. The diameter of the catheter 410 may be smaller than the diameter of the guide catheter 404 such that the catheter 410 may be slidably advanced with a lumen of the guide catheter 404. An exemplary variation of a suitable guide catheter is described in further detail below, with reference to FIG. 6.

In use, the guidewire positioning device 400 may be prepared and used for positioning of a guidewire as follows. A user may prepare the guidewire positioning device 400 at least in part by loosening the hemostasis valve 409 and sliding the inner shaft 420 in a proximal direction (e.g., to the configuration shown in FIG. 4B) until the inner shaft 420 reaches it proximal limit of movement due to the stop 430. The catheter lumen and inner shaft lumen may be flushed by filling the catheter 410 and inner shaft 420 with saline or other suitable fluid via the flush tube 407. For example, fluid may flow through the flush tube 407, into the hub 408, and into the catheter 410 and/or inner shaft 420 through one or more slots 428.

A suitable guidewire (e.g., J-tip guidewire) may then be inserted into the guidewire introducer 460, and advanced until the distal tip of the guidewire is extending beyond the distal end of the catheter 410 by a predetermined offset distance. For example, in some variations the predetermined offset distance (d) may be about 3 cm or less. The predetermined offset distance (d) may be confirmed through fluoroscopy (e.g., viewing and measuring distance between radiopaque marker(s) and/or other features of the device), echocardiographic imaging or other suitable imaging methods, through distance marker(s) at the proximal end of the guidewire relative to a proximal portion of the device (e.g., collet nut 444), etc. After the guidewire introducer is removed, the collet nut 444 may be tightened (e.g., by rotation) to secure the guidewire to the inner shaft handle 442. Accordingly, the handle 442 may be used to advance and/or torque the secured guidewire. However, when the device 400 is in the configuration shown in FIG. 4B (in which the inner shaft 420 is at its proximal limit), the secured guidewire can only be advanced distally (that is, without applying force sufficient to overcome the stop 430). In other words, the guidewire secured in this manner is substantially prevented from being withdrawn proximally into the catheter lumen. Moreover, the guidewire is substantially prevented from being moved to a position where the distance between its distal end and the distal end of the catheter 410 is less than the predetermined offset distance.

The introducer assembly 402 may be moved distally down the device 400 (e.g., down the catheter 410), and the distal ends of the catheter 410 and the guidewire may be withdrawn into the introducer assembly 402. The introducer assembly 402 may then be inserted into a portion of the guide catheter 404 (e.g., a hub of the guide catheter 404, not pictured), and one or more valves may be opened to allow any trapped air to bleed out of the introducer assembly 402 through the prep tube 406. The device 400 (e.g., catheter 410) may be advanced through the guide catheter to a desired distance, and the prep tube 406 may be removed from the assembly.

The guidewire positioning device 400 may then be advanced through the guide catheter to facilitate the placement of a guidewire along the ventricular wall regions where the device is to be implanted (e.g., around at least a portion of the circumference of the left ventricle), and/or between the chordae tendineae and the endocardium. In some variations, the advancement of the guidewire positioning device 400 through the guide catheter may be performed under fluoroscopic guidance, echocardiographic imaging, and/or other suitable imaging methods. Furthermore, during advancement of the guidewire positioning device 400, the distal end of the catheter 410 may be torqued such that it is pointing toward the mitral valve.

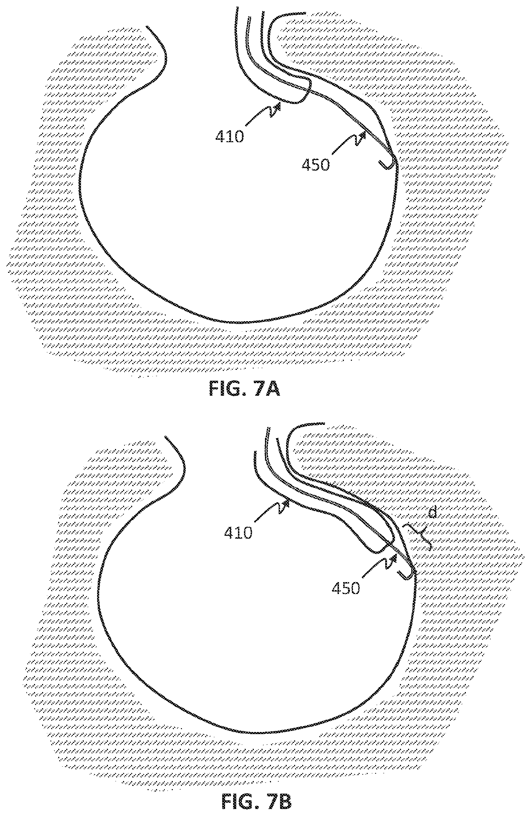

As shown in FIG. 7A and 7B, the inner shaft handle 442 may be moved distally forward (thereby advancing the guidewire 450 distally forward) until an obstruction (e.g., trabeculations, muscle bands, or other obstacles along the left ventricular wall) is encountered. For example, as shown in FIG. 7A, the guidewire 450 may encounter the ventricular wall tissue. Once the obstruction is encountered, the inner shaft handle 442 is held steady to hold the guidewire 450 in place, while the catheter handle 412 is moved distally forward (thereby advancing the catheter 410 forward) until the distal end of the catheter 410 is just proximal to the obstruction (or the distance between the distal ends of the catheter 410 and guidewire 450 is reduced to the predetermined offset distance (d) (FIG. 7B)). If the inner shaft 420 is not yet at its proximal limit of travel after the catheter 410 is advanced over the guidewire 450 in this manner, then the guidewire 450 may be further withdrawn proximally until the inner shaft 420 is at its proximal limit of travel. The processes shown in FIG. 7A and 7B may be repeated to alternately advance the guidewire 450 and the catheter 410 to track along at least a portion of the circumference of the left ventricular wall. In some variations, the guidewire 450 and/or the catheter 410 may be positioned along the subannular groove such that the distal tip of the guidewire exits the outflow tract and optionally re-crosses the aortic arch. Accordingly, the tip of the catheter may be manipulated to navigate the guidewire around one or more various obstructions during guidewire advancement. In some variations, the guidewire 450 and/or the catheter 410 may be positioned lower than the mitral valve plane or annulus, such down to or along the papillary muscle insertion on the ventricle wall (e.g., down to about 25 to about 30 mm below the mitral valve plane).

Furthermore, the position of the guidewire 450 may be confirmed (e.g., using fluoroscopy, echocardiographic imaging, and/or other suitable imaging methods). Once the guidewire 450 and/or the device 400 is positioned in the desired location, the guidewire 450 may then be unlocked from the inner shaft 410, thereby allowing separation of the guidewire 450 and the device 400. In some variations, the device 400 may be proximally withdrawn over the placed guidewire 450, thereby leaving the guidewire 450 in place to facilitate the positioning of other catheters and devices in the left ventricle. In other variations, the guidewire 450 may be withdrawn from the placed device 400 and replaced by a second guide 450 (e.g., which may be of another suitable size for facilitating the positioning of other catheters and devices in the left ventricle).

Thus, as described herein, the guidewire positioning device 400 of using the same may facilitate positioning of a guidewire in a manner that is safer and easier to use than other conventional devices. For example, the devices described herein substantially prevent the guidewire from being inadvertently withdrawn into the catheter, which would otherwise interfere with the positioning process.

### Kits

Also described herein are kits comprising a guidewire positioning device and one or more guidewires. The guidewire positioning device may comprise a catheter comprising a catheter lumen, an inner shaft arranged at least partially within the catheter lumen and comprising a shaft lumen configured to receive the guidewire, and a locking mechanism configured to lock the guidewire relative to the inner shaft to maintain at least a predetermined longitudinal offset distance between the guidewire and the catheter. The inner shaft and the catheter may be engageable so as to limit proximal longitudinal movement of the inner shaft within the catheter lumen.

In some variations, a kit may further comprise a guide catheter. FIG. 6 depicts an exemplary variation of a guide catheter 601 in which the guidewire positioning device may be advanced during use in placing a guidewire. The guide catheter 601 may comprise an elongate body 603 and a distal portion 605 comprising one or more pre-shaped curves 607. The pre-shaped curves 607 may have contours and/or bends that correspond to the contours and/or bends of a patient's vasculature such that advancing and/or aligning the guide catheter along the contours and/or bends of the patient's vasculature automatically positions the distal-most end 609 of the guide catheter at or near the mitral valve annulus and/or subannular groove region in the left ventricle. In some variations, the distal portion 605 of the guide catheter 601 may comprise a steerable, deflectable tip portion which may allow the curvature of the distal portion to be adjusted (e.g., by using a deflection knob on a proximal handle of the guide catheter).

In some variations, a kit may further comprise one or more implants for reshaping (e.g., reverse-remodeling) a heart ventricle, and/or one or more devices for delivering the same. The implant and/or devices for delivering the implant may be configured for advancement over the guidewire (or another guidewire which may be positioned using the guidewire positioning device). For example, the kit may comprise an implant that may be implanted in a left ventricle in need of reshaping, such as implants described in U.S. Patent No. 8,343,173 and U.S. Patent No. 8,641,727. Furthermore, the kit may comprise a multi-window catheter such as that described in U.S. Patent No. 8,790,367 and U.S. Patent Application Publication No. 2018/0154111, lock devices such as those described in U.S. Patent No. 9,636,106, cinching and/or locking catheters such as those described in U.S. Patent No. 8,795,298 and U.S. Patent Application Publication No. 2018/0140421, and/or cutting catheters such as those described in U.S. Provisional Patent Application Serial No. 62/847,279 filed July 17, 2019, and/or another suitable anchor delivery catheter for use in delivering anchors for the implant. The kit may further comprise any suitable delivery catheters and/or other implant devices, etc.

The foregoing description, for purposes of explanation, used specific nomenclature to provide a thorough understanding of the invention. However, it will be apparent to one skilled in the art that specific details are not required in order to practice the invention. Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described in order to best explain the principles of the invention and its practical applications, they thereby enable others skilled in the art to best utilize the invention and various embodiments with various modifications as are suited to the particular use contemplated. It is intended that the following claims define the scope of the invention.

## Claims

1. A device (100; 400) for positioning a guidewire, the device comprising:
a catheter (110; 310; 410) comprising a catheter lumen (116; 316);
an inner shaft (120; 320; 420) arranged at least partially within the catheter lumen and comprising a shaft lumen (126; 326) configured to receive a guidewire (350; 450), wherein the inner shaft and the catheter are engageable so as to limit proximal longitudinal movement of the inner shaft within the catheter lumen; and
a locking mechanism (140; 340; 440) configured to lock the guidewire to the inner shaft to maintain at least a predetermined longitudinal offset distance between the guidewire and the catheter.

2. The device of claim 1, wherein the inner shaft comprises a first feature and the catheter comprises a second feature, and wherein the first feature and the second feature are engageable so as to define a proximal limit of longitudinal movement of the inner shaft within the catheter lumen.

3. The device of claim 2, wherein at least one of the first feature and the second feature comprises a stop.

4. The device of claim 2, wherein the first feature comprises a first projection extending radially outward and wherein the second feature comprises a second projection extending radially inward.

5. The device of claim 4, wherein the first projection comprises a ring arranged around a portion of the inner shaft, and wherein the second projection comprises a wall configured to abut the ring.

6. The device of claim 2, wherein the catheter comprises a catheter housing comprising the second feature.

7. The device of claim 1, wherein the locking mechanism comprises a shaft housing coupled to the inner shaft, wherein the device optionally further comprises a collet configured to tighten the shaft housing around the inner shaft and the guidewire.

8. The device of claim 1, wherein a distal end of the catheter is curved.

## Patentansprüche

1. Vorrichtung (100; 400) zum Positionieren eines Führungsdrahts, wobei die Vorrichtung Folgendes umfasst:
einen Katheter (110; 310; 410), der ein Katheterlumen (116; 316) umfasst,
einen inneren Schaft (120; 320; 420), der mindestens teilweise in dem Katheterlumen angeordnet ist und ein Schaftlumen (126; 326) umfasst, das zur Aufnahme eines Führungsdrahts (350; 450) ausgestaltet ist, wobei der innere Schaft und der Katheter in Eingriff bringbar sind, um eine proximale Längsbewegung des inneren Schafts in dem Katheterlumen zu begrenzen, und
einen Verriegelungsmechanismus (140; 340; 440), der dazu ausgestaltet ist, den Führungsdraht an dem inneren Schaft zu verriegeln, um mindestens einen vorbestimmten Längsversatzabstand zwischen dem Führungsdraht und dem Katheter aufrechtzuerhalten.

2. Vorrichtung nach Anspruch 1, wobei der innere Schaft ein erstes Merkmal umfasst und der Katheter ein zweites Merkmal umfasst und wobei das erste Merkmal und das zweite Merkmal in Eingriff bringbar sind, um eine proximale Grenze der Längsbewegung des inneren Schafts in dem Katheterlumen zu definieren.

3. Vorrichtung nach Anspruch 2, wobei das erste Merkmal und/oder das zweite Merkmal einen Anschlag umfassen.

4. Vorrichtung nach Anspruch 2, wobei das erste Merkmal einen ersten Vorsprung umfasst, der sich radial nach außen erstreckt, und wobei das zweite Merkmal einen zweiten Vorsprung umfasst, der sich radial nach innen erstreckt.

5. Vorrichtung nach Anspruch 4, wobei der erste Vorsprung einen Ring umfasst, der um einen Abschnitt des inneren Schafts angeordnet ist, und wobei der zweite Vorsprung eine Wand umfasst, die zum Anliegen an dem Ring ausgestaltet ist.

6. Vorrichtung nach Anspruch 2, wobei der Katheter ein Kathetergehäuse umfasst, das das zweite Merkmal umfasst.

7. Vorrichtung nach Anspruch 1, wobei der Verriegelungsmechanismus ein Wellengehäuse umfasst, das an die innere Welle gekoppelt ist, wobei die Vorrichtung optional ferner eine Spannzange umfasst, die dazu ausgestaltet ist, das Wellengehäuse um den inneren Schaft und den Führungsdraht herum festzuziehen.

8. Vorrichtung nach Anspruch 1, wobei ein distales Ende des Katheters gekrümmt ist.

## Revendications

1. Dispositif (100 ; 400) servant au positionnement d'un fil-guide, le dispositif comprenant :
un cathéter (110 ; 310 ; 410) comprenant une lumière de cathéter (116 ; 316) ;
une tige interne (120 ; 320 ; 420) disposée au moins partiellement à l'intérieur de la lumière du cathéter et comprenant une lumière de tige (126 ; 326) conçue pour recevoir un fil-guide (350 ; 450), la tige interne et le cathéter pouvant être mis en prise de manière à limiter le mouvement longitudinal proximal de la tige interne à l'intérieur de la lumière du cathéter ; et
un mécanisme de verrouillage (140 ; 340 ; 440) conçu pour verrouiller le fil-guide relativement à la tige interne afin de maintenir au moins une distance de décalage longitudinale prédéterminée entre le fil-guide et le cathéter.

2. Dispositif selon la revendication 1, dans lequel la tige interne comprend un premier élément et le cathéter comprend un second élément, et dans lequel le premier élément et le second élément peuvent être mis en prise de manière à définir une limite proximale de mouvement longitudinal de la tige interne à l'intérieur de la lumière du cathéter.

3. Dispositif selon la revendication 2, dans lequel au moins l'un du premier élément et du second élément comprend une butée.

4. Dispositif selon la revendication 2, dans lequel le premier élément comprend une première saillie s'étendant radialement vers l'extérieur et dans lequel le second élément comprend une seconde saillie s'étendant radialement vers l'intérieur.

5. Dispositif selon la revendication 4, dans lequel la première saillie comprend un anneau placé autour d'une partie de la tige interne, et dans lequel la seconde saillie comprend une paroi conçue pour venir buter contre l'anneau.

6. Dispositif selon la revendication 2, dans lequel le cathéter comprend une enveloppe de cathéter comprenant le second élément.

7. Dispositif selon la revendication 1, dans lequel le mécanisme de verrouillage comprend une enveloppe de tige accouplée à la tige interne, dans lequel le dispositif comprend en outre facultativement une pince de serrage conçue pour serrer l'enveloppe de tige autour de la tige interne et du fil-guide.

8. Dispositif selon la revendication 1, dans lequel une extrémité distale du cathéter est incurvée.
